# EUROPEAN PATENT SPECIFICATION

(11) **EP 0 423 289 B1**
(45) Date of publication and mention of the grant of the patent: **01.12.1993**
(21) Application number: 90906592.2
(22) Date of filing: 17.04.1990
(51) Int. Cl.: A61M 5/14, A61M 5/168

(54) **COMPOSITION OF BENEFICIAL AGENT AND METHOD FOR ADMINISTERING THE SAME BY CONTROLLED DISSOLUTION**
ZUSAMMENSETZUNG EINES HEILMITTELS UND VERFAHREN ZUR VERABREICHUNG BEI EINER KONTROLLIERTEN LÖSUNGSGESCHWINDIGKEIT
COMPOSITION D'UN AGENT UTILE ET METHODE D'ADMINISTRATION DE CELUI-CI PAR DISSOLUTION CONTROLEE

(30) Priority: 01.05.1989 US 345334
(43) Date of publication of application: 24.04.1991
(73) Proprietor: BAXTER INTERNATIONAL INC., Deerfield, IL 60015 (US)
(72) Inventor: BREILLATT, Julian, P., Jr., Mundelein, IL 60060 (US); WOO, Lecon, Libertyville, IL 60048 (US); NELSON, Deanna, J., Libertyville, IL 60048 (US); APPL, Richard, Downers Grove, IL 60516 (US); WEINLESS, Naomi, L., Highland Park, IL 60035 (US); POKROPINSKI, Sharon, Berwyn, IL 60402 (US); SOLTYS, Paul, Palatine, IL 60074 (US); BARENBERG, Sumner, A., Chicago, IL 60611 (US)
(74) Representative: MacGregor, Gordon
(86) International application number: US9002053
(87) International publication number: WO9013323

(56) References cited:
- EP-A- 0 059 694
- WO-A-86/00004
- WO-A-89/04639
- WO-A-89/04654
- WO-A-89/04687
- WO-A-89/04689
- WO-A-90/4688
- FR-A- 2 510 413

## Description

### FIELD OF THE INVENTION

The present invention relates to delivery of beneficial agents to flowing medical fluids. More specifically, this invention pertains to predetermined, controlled and variable delivery of beneficial agents to a flowing medical fluid, which agents may be otherwise insoluble or unstable in the medical fluid.

### BACKGROUND OF THE INVENTION

Parenteral delivery and collection of medical fluids are important factors in current medical care. Such fluids are delivered principally via intravenous and intraperitoneal routes, while phlebotomy constitutes the major collection modality. Intravenous fluids commonly include blood and blood fractions, sugar, electrolyte and osmotic solutions, and nutrient preparations. Fluids are generally delivered intraperitoneally to remove excreted nitrogenous wastes from end stage renal disease patients in a process known as peritoneal dialysis. Many beneficial and therapeutic agents are preferably delivered via parenteral fluids to avoid digestive tract and liver associated modification of those agents. Historically such agents have been formulated and added to the parenteral fluid reservoir by a pharmacist or nurse. Because this is a labor intensive step with opportunity for error, and because many beneficial agents are less stable in solution than in dry form, systems have been developed to facilitate formulation of soluble dry agents with parenteral fluid immediately before use. One such system disclosed in U.S. Pat. No. 4,614,267 still requires manual formulation.

The next advance provided preprogrammed, unattended systems to formulate parenteral fluid with soluble dry agents in situ in a formulation chamber associated with the primary delivery set. An example of such a system is disclosed in U.S. Pat. 4,552,555 in which the beneficial agent is contained within a formulation chamber bounded by a membrane. The membrane serves to control the delivery rate of beneficial agent into the flowing stream of medical fluid. Controlled delivery of a beneficial agent into a medical fluid can also be achieved through diffusion of the agent from a non-erodible polymer matrix that contains and serves as a reservoir of the agent. Generally the permeability of the matrix controls the diffusion rate of the agent into the surrounding environment. Such systems are described in U.S. Pat. Nos. 3,921,636 and 4,511,353. When a membrane or matrix system is used to control beneficial agent delivery into parenteral fluids, actual delivery rate of the agent to the patient is substantially independent of fluid flow rate; a benefit if adequate fluid flow control means are not available. However, that same flow rate independence restricts the ability of the healthcare professional to increase dose rate to meet patient needs. While such inventions have improved the efficiency and safety of administering beneficial agents to parenteral fluids, they fall short of the ideal: (1) by not providing a clear visual indication that the dose has been delivered; 2) by causing a major portion of the beneficial agent in the formulation chamber to dissolve in the parenteral fluid early in the delivery procedure, thereby leaving the agent in solution for the extended period of time required to deliver the dose; and, (3) by requiriny that the agent for formulation be present in a form which is soluble in the parenteral fluid.

The ideal system for parenteral or blood delivery would be a matrix that contains and/or protects, as necessary, one or more entrapped beneficial agents in a dry, stable state; that, when placed in a flow chamber in the medical fluid administration line, disappears as the agent is delivered into the flowing stream, indicating delivery of the dose. The simplest means of effecting disappearance is dissolution of the matrix in the stream of medical fluid, thereby releasing the entrapped agent.

This suggests the matrix should be formed from innocuous components clinically acceptable for use in parenteral fluids and could be the beneficial agent itself or a component thereof, a beneficial metabolite, or a non-toxic excretable molecule.

Preferably, the matrix would dissolve proportionally to the flow of medical fluid over its surface with its dissolution rate determining the delivery rate of the beneficial agent; thus allowing the beneficial agent to remain stable and protected within the matrix until both matrix and agent become hydrated and dissolve. The dissolution rate of the beneficial agent should match or exceed the dissolution rate of the matrix; otherwise the matrix could dissolve away leaving the beneficial agent as an insoluble mass in the flowing fluid.

Since the most stable forms of many drugs are not directly soluble in water or saline, it would be advantageous to use them as the storage form in the matrix, co-entrapped with dry modifying agents, such as pH buffers or solubilizing reactants, under conditions that they neither react with each other during the formation of the matrix nor during storage, but such that when the dissolviny fluid reaches the beneficial agent, it will have dissolved sufficient pH buffer or reactant to create a localized environment in the fluid boundary layer that favors dissolution of the otherwise insoluble beneficial agent.

A similar rationale leads to matrix compositions that deliver active agents from inactive storage forms co-entrapped with their activator species; or, as examples of other variations, beneficial agents in their active forms co-entrapped with inhibitors of enzymes that would inactivate the agents, and, initial release of a beneficial agent followed in time by the release of its inactivator. The latter case may be achieved, for example, by forming the matrix with the separate agents in defined concentric layers. The matrix may also serve as a separator of mutually reactive species. In such a case, the matrix must be appropriately inert in a chemical sense towards the beneficial agents and the reactants associated with them.

In summary, controlled dissolution operates at five different levels to provide controlled administration of beneficial agents to flowing medical fluids: (1) the flow field of the medical fluid in conjunction with the shape of the solid matrix element, (2) the dissolution properties of the matrix material, (3) the geometric distribution pattern of beneficial agent and modifyiny agent particles within the matrix, (4) the dissolution properties and size of beneficial agent and modifying agent particles, and (5) the chemical/solubilizing interactions between beneficial agents and modifying agents at the solid/fluid interface.

While each beneficial agent or combination of such agents may require individual adjustment for optimum matrix composition and method of formation, general principles are available. It is well known that essential oils with extreme sensitivity to heat, light, air and moisture can be stabilized for storage in dry form by encapsulation in dry sugar melts using methods disclosed by Swisher in U.S Pat. Nos. 2,809,895 and 3,041,180. Similarly, therapeutic activity of heat sensitive beneficial agents can be preserved by incorporating them at moderate temperatures into a cooling melt of the embedding matrix using standard candy manufacture methods such as those disclosed by Mozda in U.S Pat. No. 4,753,800. Sair, in choosing a protective and stabilizing matrix material for encapsulated food additives, disclosed in U.S Pat. No. 4,232,047 that mustard oil was less reactive with a matrix composed of polymeric carbohydrate than with a protein matrix. Analogous reasoning when choosing initial matrix materials for parenteral fluids might suggest use of non-reducing monomeric carbohydrates. Water may be the most chemically active molecule in a matrix, and stability of certain beneficial agents could require rigorous exclusion of reactive water from their encapsulating matrix, even below the 0.5 to 2.0% water content values considered virtually anhydrous by Swisher in U.S. Pat. No. 3,041,180.

WO-A-8904689, WO-A-8904687, WO-A-8904654, WO-A-8904688 and WO-A-8904639, all of which are publications within the meaning of Art. 54(3) EPC, disclose methods for controlled administration of a beneficial agent to a medical fluid flowing over a solid body comprising a matrix with the agent distributed uniformly throughout the matrix.

Claims 1 and 7 are based in their precharacterising parts on WO-A-8904687, and the distinguishing features of the present invention are set out in the characterising parts of Claims 1 and 7.

When the matrix contains two agents, the agents may also be dispersed in such a way as to isolate agents from one another until the moment of release into the fluid. This is desireable in situations such as, for example; (1) where one might wish to isolate mutually reactive agents from one another; or, (2) store an agent in the matrix in an insoluble, inactive form and include within the matrix a solubilizer and activator which, upon dissolution of the matrix, will solubilize and activate the agent as it is being administered.

### DESCRIPTION OF THE INVENTION

There is provided provides an anhydrous, solid, water-soluble, particulate composition for dissolution-controlled administration of one or two beneficial agents to a medical fluid. It includes a matrix, preferably comprising one or more sugars or sugar alcohols in anhydrous solidified melt form. The beneficial agent or agents are distributed in particulate form and in predetermined pattern within the matrix. Where the matrix contains two agents, one agent may be a Chemical species that modifies the other beneficial agent or modulates its action or delivery. Fabrication methods may be chosen such that each particle is surrounded by, and isolated from other particles by the continuous phase of the matrix substance.

For use this composition is made into solid formed elements whose external shape and geometry partially determine the relative dissolution rate of their various surfaces in a flowing stream of medical fluid. The disappearance of the composition provides a visual indicator of delivery of the beneficial agent into the stream of medical fluid.

The present invention allows the healthcare practitioner a tremendous amount of flexibility in delivering parenterally to a patient agents which were heretofore otherwise difficult or impossible to deliver because they were insoluble, had very limited shelf-life, were incompatible with one another, etc.

More specifically, the present invention tremendously simplifies the parenteral administration of
a beneficial agent in a predetermined manner by distributing the agent in the matrix in a specific geometric pattern whose proximity to the dissolving edge of the solid element determines the time of its dissolution and its time-based concentration profile in the flowing medical fluid.
In a preferred embodiment, a first, easily inactivated, beneficial agent may be stored in the matrix along with a second agent which is a modulating species, which upon dissolution into the medical fluid acts to extend the active lifetime of the first beneficial agent by inhibiting potentially inactivating enzymes or factors present in the medical fluid and/or body fluid to which the first agent is ultimately delivered.
In another preferred embodiment, the matrix may contain a beneficial agent and its inactivator or its antagonits. The beneficial agent and its inactivator or its antagonist may be stored in the matrix in separate particulate form and in specific geometric distributions that provide sequential release into the flowing stream of medical fluid, which allows the beneficial agent to re-ach its site of action and act, then either be inactivated or have its action modulated by its antagonist before the beneficial agent can act on other sites where its action is undesirable.

Both the fine structure of the particulate composition and the shape of the formed element play important roles in its delivery function by providing a dynamic formulation and reaction region that includes the solid matrix/fluid boundary layer interface where insoluble and/or inactive beneficial agent precursors may be transformed into pharmaceutically acceptable forms in an unattended, predetermined manner not requiring artificial diffusion limiting devices such as membranes or polymeric matrices.

By example, the flow of medical fluid past the surface of the formed element generally establishes an unmixed fluid boundary layer at the solid/fluid interface. As the particulate composition dissolves in the fluid, the matrix components, beneficial agent or agents accumulate in the boundary layer, then diffuse into the flowing stream. Chemical reactions previously prevented by physical separation of particulates in the solid matrix now occur in this concentrated boundary layer, both between two soluble species and between insoluble species exposed at the solid interface by dissolution of the matrix and soluble species.

The particulate composition of this invention preferably may be manufactured by dispersion of a dry, powdered beneficial agent into a dry, water soluble fluid phase material and thereafter allowing the dispersion thus formed to assume a solid state. The fluid phase material shall preferably include a molten sugar or a mixture of molten sugars, and optionally, soluble, heat stable, chemical species compatible with the included particulate beneficial agent. The powdered or particulate beneficial agent is preferably dispersed in the fluid phase material after it has cooled from a melt, but while it still retains sufficient fluidity to allow inclusion of the particles. Dispersion of the beneficial agent may be effected by mixing in its various forms, or preferably by well known cooker-extruder processes wherein one may pass directly from a dry material to a final product containing a predetermined geometric distribution of the beneficial agent within the body of the solid formed elements.

Vigorous mixing of powdered admixtures into fluid cooling melts generally causes each solid particle to be coated with the continuous fluid phase, thereby isolating the various particles from each other. Moreover, dry powdered mixtures of mutually reactive beneficial agents generally may be embedded in the dry, fluid cooling melt without reaction. In a case where it is necessary that the mutually reactive beneficial agents be rigorously excluded from contacting each other, they may be admixed separately and sequentially into the cooling melt.

The term sugar shall include, singly and in combination, monosaccharides or polymers and derivatives thereof having a degree of polymerization of preferably about 1 or 2, but optionally up to the hundreds. The term sugar also includes, singly and in combination, the corresponding sugar alcohols and polymers thereof. Preferably, a sugar for use herein may include one or a combination of glucose, fructose, mannose, lactose, sucrose, trehalose, sorbitol, mannitol, xylitol, glycerol, lycasin, dextran, starch, hydroxyethyl starch, and the like.

The solidified melt of sugars that constitutes the matrix of the particulate composition may assume various physical forms depending on the combination of sugars used, the conditions of preparation, and the nature of the particulate inclusions embedded therein. It is expected that glassy, amorphous non-crystalline regions may occur in conjunction with both bulk macrocrystalline and microcrystalline regions. Where predominantly glassy morphology is desired, bulk sugars such as sorbitol and glucose may be plasticize by agents such as xylitol, glycerol or water.

Substantially anhydrous matrix composition shall be understood to mean less than 2% water content and preferably less than 1% water. However, for certain beneficial agents that are unstable in the presence of even small amounts of water, matrix compositions containing about 0.1% water are readily achievable and employed.

Specific examples of methods by which solid sugar matrix material may be prepared for inclusion of particulate benefical agents include boiling sugar solutions to such temperatures as is required to achieve less than 1% water content, or by melting sugars with or without a plasticizing agent such as water or glycerol. For example, a sorbitol solution boiled to 195°C at atmospheric pressure contains about 1% water; whereas anhydrous sorbitol melted in an oven at 150°C alone or with 1% anhydrous glycerol can contain about 0.1% water content.

## Claims

1. A method for controlled administration of a beneficial agent to a flowing medical fluid wherein the fluid is passed over a solid body comprising a matrix that contains the beneficial agent, characterised in that the matrix has a predetermined rate of dissolution in said fluid corresponding to the rate of flow of said fluid, and in that the beneficial agent is not uniformly distributed in the matrix, but is distributed in the matrix in a geometric pattern within the solid body so that the time-based concentration profile of the agent in the flowing medical fluid is determined by the proximity of the pattern to the dissolving edge of the solid body.

2. A method according to Claim 1, wherein the matrix is in the form of a substantially anhydrous solid composition, whereby the beneficial agent is delivered to the medical fluid at a predetermined rate as said matrix dissolves.

3. The method of Claim 1 or 2 wherein a second beneficial agent is distributed in said matrix such that said second agent will be delivered directly into the flow of said fluid at a predetermined rate as said matrix dissolves.

4. The method of Claim 3 wherein said second beneficial agent is distributed in said matrix in a geometric pattern.

5. The method of Claim 4 wherein the second beneficial agent is distributed in said matrix in such a way as to be isolated from said first beneficial agent.

6. The method of Claim 3, 4 or 5 wherein said first and second beneficial agents are delivered into the flow of said medical fluid sequentially.

7. An anhydrous, solid composition for use in controlled administration of a beneficial agent to a flowing medical fluid and comprising a solid body comprising a matrix that contains the beneficial agent, characterised in that the matrix has a predetermined rate of dissolution in said fluid related to the rate of flow of the fluid, in that the beneficial agent is not uniformly distributed in the matrix, but is distributed in the matrix in a geometric pattern within the solid body so that the time-based concentration profile of the agent in the flowing medical fluid is determined by the proximity of the pattern to the dissolving edge of the solid body.

8. The anhydrous composition of Claim 7 wherein a second beneficial agent is distributed in the matrix for delivery as the matrix dissolves.

9. The anhydrous composition of Claim 8 wherein the second agent is a modifier agent for the first agent as the agents are being administered.

10. The anhydrous composition of Claim 9 wherein said first agent is distributed in said matrix in an insoluble form and said second agent is a solubilizing agent of said first agent.

11. The anhydrous composition of Claim 9 wherein said first agent is distributed in said matrix in an inactive form and said second agent is an activating agent of said first agent.

12. The anhydrous composition of Claim 9 wherein said first agent is distributed in said matrix in an active form and said second agent is an antagonist of said first agent.

13. The anhydrous composition of Claim 9 wherein said first agent is distributed in said matrix in an active form and said second agent is an inactivator of said first agent.

14. The anhydrous composition of Claim 9 wherein said first agent is distributed in said matrix in an active form and said second agent is an inhibitor of an enzyme or factor which reacts with said first agent.

15. The anhydrous composition of any one of Claims 8 to 14 wherein said second beneficial agent is distributed in said matrix in a geometric pattern.

16. The anhydrous composition of any one of Claims 8 to 15 wherein said second agent is dispersed in said matrix in such a way as to be isolated from said first agent.

17. A method according to Claim 1, wherein the solid composition has the features of any one of Claims 7 to 16.

## Patentansprüche

1. Verfahren zur kontrollierten Verabreichung eines gesundheitsfördernden Mittels an ein strömendes medizinisches Fluid, wobei das Fluid über einen festen Körper geleitet wird, der eine Matrix aufweist, die das gesundheitsfördernde Mittel enthält,
dadurch gekennzeichnet,
daß die Matrix eine vorbestimmte Auflösungsrate in dem Fluid hat, die der Durchflußrate des Fluids entspricht, und daß das gesundheitsfördernde Mittel in der Matrix nicht gleichmäßig verteilt ist, sondern in der Matrix in einem geometrischen Muster innerhalb des festen Körpers verteilt ist, so daß das zeitbezogene Konzentrationsprofil des Mittels in dem strömenden medizinischen Fluid durch die Nähe des Musters zu dem sich auflösenden Rand des festen Körpers bestimmt wird.

2. Verfahren nach Anspruch 1,
wobei die Matrix in Form einer im wesentlichen wasserfreien festen Zusammensetzung vorliegt, so daß das gesundheitsfördernde Mittel mit einer vorbestimmten Rate an das medizinische Fluid abgegeben wird, während sich die Matrix auflöst.

3. Verfahren nach Anspruch 1 oder 2,
wobei ein zweites gesundheitsförderndes Mittel in der Matrix derart verteilt ist, daß das zweite Mittel mit einer vorbestimmten Rate direkt in den Strom des Fluids abgegeben wird, während sich die Matrix auflöst.

4. Verfahren nach Anspruch 3,
wobei das zweite gesundheitsfördernde Mittel in der Matrix in einem geometrischen Muster verteilt ist.

5. Verfahren nach Anspruch 4,
wobei das zweite gesundheitsfördernde Mittel in der Matrix auf eine solche Weise verteilt ist, daß es von dem ersten gesundheitsfördernden Mittel isoliert ist.

6. Verfahren nach Anspruch 3, 4 oder 5,
wobei das erste und das zweite gesundheitsfördernde Mittel sequentiell in den Strom des medizinischen Fluids abgegeben werden.

7. Wasserfreie feste Zusammensetzung zur Verwendung bei der kontrollierten Verabreichung eines gesundheitsfördernden Mittels an ein strömendes medizinisches Fluid, wobei die Zusammensetzung einen festen Körper mit einer Matrix aufweist, die das gesundheitsfördernde Mittel enthält, dadurch gekennzeichnet,
daß die Matrix eine vorbestimmte Auflösungsrate in dem Fluid hat, die mit der Durchflußrate des Fluids in Beziehung steht,
daß das gesundheitsfördernde Mittel in der Matrix nicht gleichmäßig verteilt ist, sondern in der Matrix in einem geometrischen Muster innerhalb des festen Körpers verteilt ist, so daß das zeitbezogene Konzentrationsprofil des Mittels in dem strömenden medizinischen Fluid durch die Nähe des Musters zu dem sich auflösenden Rand des festen Körpers bestimmt ist.

8. Wasserfreie Zusammensetzung nach Anspruch 7,
wobei ein zweites gesundheitsförderndes Mittel in der Matrix verteilt ist, um abgegeben zu werden, während sich die Matrix auflöst.

9. Wasserfreie Zusammensetzung nach Anspruch 8,
wobei das zweite Mittel ein Modifikationsmittel für das erste Mittel ist, während die Mittel verabreicht werden.

10. Wasserfreie Zusammensetzung nach Anspruch 9,
wobei das erste Mittel in der Matrix in einer unlöslichen Form verteilt ist und das zweite Mittel ein Lösungsvermittler für das erste Mittel ist.

11. Wasserfreie Zusammensetzung nach Anspruch 9,
wobei das erste Mittel in der Matrix in einer inaktiven Form verteilt ist und das zweite Mittel ein Aktivierungsmittel für das erste Mittels ist.

12. Wasserfreie Zusammensetzung nach Anspruch 9,
wobei das erste Mittel in der Matrix in einer aktiven Form verteilt ist und das zweite Mittel ein Antagonist des ersten Mittels ist.

13. Wasserfreie Zusammensetzung nach Anspruch 9,
wobei das erste Mittel in der Matrix in einer aktiven Form verteilt ist und das zweite Mittel ein Inaktivierungsmittel für das erste Mittel ist.

14. Wasserfreie Zusammensetzung nach Anspruch 9,
wobei das erste Mittel in der Matrix in einer aktiven Form verteilt ist und das zweite Mittel ein Inhibitor eines Enzyms oder Faktors ist, das/der mit dem ersten Mittel reagiert.

15. Wasserfreie Zusammensetzung nach einem der Ansprüche 8 bis 14,
wobei das zweite gesundheitsfördernde Mittel in der Matrix in einem geometrischen Muster verteilt ist.

16. Wasserfreie Zusammensetzung nach einem der Ansprüche 8 bis 15,
wobei das zweite Mittel in der Matrix auf eine solche Weise dispergiert ist, daß es von dem ersten Mittel isoliert ist.

17. Verfahren nach Anspruch 1,
wobei die feste Zusammensetzung die Merkmale nach einem der Ansprüche 7 bis 16 hat.

## Revendications

1. Méthode pour l'administration contrôlée d'un agent utile dans un fluide médical en circulation, dans laquelle on fait passer le fluide sur un corps solide comprenant une matrice qui contient l'agent utile, caractérisée en ce que la matrice a une vitesse de dissolution prédéterminée dans ledit fluide, correspondant au débit de ce fluide, et en ce que l'agent utile n'est pas uniformément réparti dans la matrice mais est réparti dans la matrice suivant une configuration géométrique à l'intérieur du corps solide de sorte que le profil de concentration en fonction du temps de l'agent dans le fluide médical en circulation est déterminé par la proximité de la configuration par rapport au bord en dissolution du corps solide.

2. Méthode suivant la revendication 1, dans laquelle la matrice est sous la forme d'une composition solide sensiblement anhydre, de sorte que l'agent utile est introduit dans le fluide médical à une vitesse prédéterminée, lorsque ladite matrice se dissout.

3. Méthode suivant la revendication 1 ou 2, dans laquelle un deuxième agent utile est réparti dans ladite matrice d'une manière telle que ledit deuxième agent est introduit directement dans l'écoulement dudit fluide, à une vitesse prédéterminée, lorsque ladite matrice se dissout.

4. Méthode suivant la revendication 3, dans laquelle le deuxième agent utile est réparti dans la dite matrice suivant une configuration géométrique.

5. Méthode suivant la revendication 4, dans laquelle le deuxième agent utile est réparti dans ladite matrice de manière à être isolé dudit premier agent utile.

6. Méthode suivant la revendication 3,4 ou 5, dans laquelle lesdits premier et deuxième agents utiles sont introduits dans ledit fluide médical en circulation, de façon séquentielle.

7. Composition solide anhydre utilisable pour l'administration contrôlée d'un agent utile dans un fluide médical en circulation et comprenant un corps solide constitué d'une matrice qui contient l'agent utile, caractérisée en ce que la matrice a une vitesse prédéterminée de dissolution dans ledit fluide en relation au débit du fluide, en ce que l'agent utile n'est pas uniformément réparti dans la matrice mais est réparti dans la matrice suivant une configuration géométrique à l'intérieur du corps solide de sorte que le profil de concentration en fonction du temps de l'agent dans le fluide médical en circulation est déterminé par la proximité de la configuration par rapport au bord en dissolution du corps solide.

8. Composition anhydre suivant la revendication 7, dans laquelle un deuxième agent utile est réparti dans la matrice pour distribution lorsque la matrice se dissout.

9. Composition anhydre suivant la revendication 8, dans laquelle le deuxième agent est un agent modificateur pour le premier agent, pendant l'administration des agents.

10. Composition anhydre suivant la revendication 9, dans laquelle ledit premier agent est réparti dans la dite matrice sous une forme insoluble et ledit deuxième agent est un agent de solubilisation dudit premier agent.

11. Composition anhydre suivant la revendication 9, dans laquelle ledit premier agent est réparti dans ladite matrice sous une forme inactive et ledit deuxième agent est un agent d'activation dudit premier agent.

12. Composition anhydre suivant la revendication 9, dans laquelle ledit premier agent est réparti dans ladite matrice sous une forme active et ledit deuxième agent est un antagoniste dudit premier agent.

13. Composition anhydre suivant la revendication 9, dans laquelle ledit premier agent est réparti dans ladite matrice sous une forme active et ledit deuxième agent est un inactivateur dudit premier agent.

14. Composition anhydre suivant la revendication 9, dans laquelle ledit premier agent est réparti dans ladite matrice sous une forme active et ledit deuxième agent est un inhibiteur d'une enzyme ou d'un facteur qui réagit avec ledit premier agent.

15. Composition anhydre suivant une quelconque des revendications 8 à 14, dans laquelle ledit deuxième agent utile est réparti dans ladite matrice suivant une configuration géométrique.

16. Composition anhydre suivant une quelconque des revendications 8 à 15, dans laquelle ledit deuxième agent est réparti dans la dite matrice de manière à être isolé dudit premier agent.

17. Méthode suivant la revendication 1, dans laquelle la composition solide possède les caractéristiques d'une quelconque des revendications 7 à 16.
